# EUROPEAN PATENT APPLICATION

(11) **EP 3 706 138 A1**
(43) Date of publication of application: **09.09.2020**
(21) Application number: 19160475.0
(22) Date of filing: 04.03.2019
(51) Int. Cl.: G16H 40/63, A61C 17/22

(54) **SYSTEM, DEVICE AND METHOD FOR OPERATION OF A PERSONAL CARE DEVICE**

(71) Applicant: Koninklijke Philips N.V., 5656 AG Eindhoven (NL)
(72) Inventor: FARRELL, Nathan, 5656 AE Eindhoven (NL); RONDA, Cornelis Reinder, 5656 AE Eindhoven (NL)
(74) Representative: de Haan, Poul Erik

(57) **Abstract**

A personal care system (100) comprising a personal care device body (2), wherein the personal care system (100) is configured to enable: the personal care device body (2) to be paired with a plurality of remote devices (4a-4h); a remote device to be selected from the plurality of remote devices (4a-4h) with which the personal care device body (2) is paired; and at least one operating parameter of the personal care device body (2) to be determined according to an attribute of the selected remote device (4a); and wherein the personal care device body (2) is configured to operate according to the at least one operating parameter.

## Description

### FIELD OF THE INVENTION

The present invention relates to a personal care system, personal care device body and remote devices.

### BACKGROUND

Personal care devices, such as oral cleaning devices, hair care devices, hair removal devices and skin care devices, are often provided with multiple modes or states of operation which can be altered by the user, thus providing a customizable experience. Such devices are often operated by several users who share the same device. The devices typically comprise one or more buttons with which each user can interact in order to set the operation of the personal care device as per their requirements. For example, a personal care device is often provided with a mode button to alter the operation setting of the personal care device or an intensity button to alter the operating power of the device.

However, there are a number of issues which arise with the provision of buttons as well as the sharing of a device between multiple users.

For example, buttons are expensive to provide due to the number of parts and their assembly, the increased complexity of the housing to incorporate buttons, and the electronics required for the button to operate. Buttons also typically provide a path for ingress of water to the interior of the personal care device, which is undesirable as this can cause problems with circuitry. Furthermore, the location of buttons on a personal care device are dictated by user ergonomics, for example the position of a user's hand during use. This in turn constrains the position of electronics within the device.

For the user of a shared device it is inconvenient to have to reconfigure the operating state of a personal care device using buttons prior to each use if their settings are different from the previous user's settings of the same device.

It is therefore desirable to address these concerns.

### SUMMARY OF THE INVENTION

To better address these concerns, according to an embodiment of a first aspect, there is provided a personal care system comprising a personal care device body, wherein the personal care system is configured to enable: the personal care device body to be paired with a plurality of remote devices; when the personal care device body is paired with the plurality of remote devices, a remote device to be selected from the plurality of remote devices with which the personal care device body is paired; and at least one operating parameter of the personal care device body to be determined according to an attribute of (or based on) the selected remote device; and wherein the personal care device body is configured to operate according to the at least one operating parameter.

Thus, the personal care device body may be paired with a plurality of remote devices. For example, the personal care device body may be linked with each of a plurality of remote devices to enable communication between the personal care device body and each of the plurality of remote devices. The personal care device body may be paired to two or more remote devices using near field technology, such as Bluetooth (using short-wavelength UHF radio waves in the ISM band from 2.400 to 2.485 GHz). The personal care device body may be paired to each of the plurality of remote devices using a Wi-Fi network, where the Wi-Fi network enables communication between the personal care device body and each of the remote devices. Communication between elements of the personal care system, for example between any of the plurality of remote devices and the personal care device, may be achieved using any known form of wireless communication, for example, by using sound waves and/or electromagnetic waves such as radio waves and/or light waves, magnetic fields, electric fields.

A remote device of the plurality of remote devices may pair with the personal care device body each time the remote device comes within range of the personal care device body, for example when the remote device is near enough to the personal care device body to be detected. A remote device may pair with the personal care device body automatically when it comes within range of the personal care device body. The remote device may pair with the personal care device body when the remote device and/or the personal care device body are prompted to pair with one another. The prompting may be by input by a user, for example by indicating to the remote device that it should initiate pairing with the personal care device body, or vice versa.

Once the personal care device body has been paired to a remote device a first time, the personal care device body may subsequently connect to the remote device without requiring prompting.

A remote device may indicate a user of the personal care device body. For example, each remote device may belong to a different user, such that the user of a personal care device body can be identified based on their remote device. The at least one operating parameter may be determined based on the identity of the user (the identity of the user may be the attribute of the selected remote device). The attribute of the selected remote device may be the identity of the remote device, or may be an operating parameter on the basis of which the personal care device body is configured to operate.

The selected remote device may be the remote device based on which the operating parameter of the personal care device body is determined. For example, the selected remote device may operate the personal care device body, whereas a remote device which is not selected may not operate the personal care device body. The operating parameter may be determined by the personal care device body and/or the selected remote device. The selected remote device may indicate to the personal care device body the at least one operating parameter. The selected remote device may enable operation of the personal care device body by indicating an operating parameter to the personal care device body. The remote device may be automatically selected, or the user may determine which of the plurality of remote devices is to be selected.

Thus, advantageously, the personal care device does not require buttons, as the personal care device can be controlled according to an attribute of a remote device.

According to a further aspect, the attribute may include an identity of the selected remote device which determines the at least one operating parameter of the personal care device body.

For example, the identity of the selected remote device may indicate a user of the personal care device body. The identity of the selected remote device may indicate the operating parameters that should be applied for a particular user. The selected remote device may indicate the preferred operating parameters of the personal care device body, for example the preferred at least one operating parameter of the personal care device body as determined by a user of the selected remote device. Thus, each time the same remote device is selected, the personal care device body may operate with at least one operating parameter which is associated with that remote device. Each of the plurality of remote devices may be associated with the same user, and/or may be associated with a particular at least one operating parameter. The association between an identity of the selected remote device and the at least one operating parameter may be stored in the personal care system, for example in the remote device and/or in the personal care device body. The personal care device body may determine the at least one operating parameter.

Thus, the personal care device may automatically set the operating parameters of the personal care device without requiring user input.

According to a further aspect, the personal care device body may be configured to transmit information to the selected remote device, the information being at least one of: information about a user, information about use of the personal care device body, information about operation of the personal care device body, information about use of a tool attached to the personal care device body, information about operation of a tool attached to the personal care device body.

For example, the personal care device body may communicate (for example, transmit) information to the selected remote device. The information may be information about a user. The communicated information may be information about use of the personal care device body, for example, information on how long the user uses the personal care device. The information may be information about operation of the personal care device body. For example, the information may be the at least one operating parameter of the personal care device. The information may be information about use of a tool attached to the personal care device body, for example, information about how a user is using the tool. The information may be information about operation of a tool attached to the personal care device body. For example, the information may be information about the operation of a tool in relation to the at least one operating parameter of the personal care device.

The communicated information may be transferred via a wireless network from the remote device to a remote storage device, in order to collect data on users of the personal care device body. The communicated information may be transferred using near field technology. The information may be stored in the remote device, or in a wireless network, for example using cloud computing. The information may be used to prompt the user on best use of the personal care device body. For example, if the personal care device body is an oral care device, such as a toothbrush, the information may relate to brushing technique of a user. The information may subsequently be used to indicate to the user how to improve their use of the personal care device body, such as brushing technique. The information may be displayed to the user via an application on the remote device. The information about a user may be information which is collected relating to a particular user, where information on the same user is collated.

According to a further aspect, the attribute may include an indication of the at least one operating parameter input by a user to an interface, and wherein the interface is comprised in the remote device.

The user may input an indication of the at least one operating parameter the first time a remote device is paired with the personal care device. This at least one operating parameter may be used as the default at least one operating parameter each subsequent time the personal care device is used. The user may input an indication of a different at least one operating parameter any subsequent time a remote device is paired with the personal care device. The user may alter a current at least one operating parameter. For example, the user may select a different operating parameter to the current operating parameter. The operating parameter may be input by a user to an interface, for example an application on a remote device such as a smart phone. Thus, the user may select the at least one operating parameter. A current operating parameter may be altered by the user indicating or inputting a different operating parameter. The interface may be an interface in the personal care device body, for example, a button or touch screen provided on the personal care device body through which the user can input an operating parameter. The interface may comprise voice activation.

Thus, the personal care device can be remotely operated.

According to a further aspect, the personal care system may be configured to determine if at least one previous operating parameter of the personal care device body has been previously determined according to an attribute of the selected remote device, and when at least one previous operating parameter has been previously determined, to determine the at least one operating parameter based on, or according to, the previous operating parameter.

For example, when a user uses a personal care device body, they may determine an operating parameter of the personal care device body such as a particular mode of operation. When the same remote device, and thus the same user, is selected, and the personal care device body is subsequently used, the operating parameter which is subsequently determined may be the same as the previously determined operating parameter, such as the same mode of operation. For example, where the personal care device body is an oral care device such as a toothbrush, the brushing mode which was previously selected may be determined, and the toothbrush may be set to the same brushing mode. Thus, the personal care device body may operate based on, or according to, a previously used operating parameter of the selected remote device, thereby automatically setting the personal care device to a setting appropriate for the user.

According to a further aspect, the remote device may be selected based on at least one of: the remote device of the plurality of remote devices which is closest in distance to the personal care device body, input by a user which indicates which remote device is to be selected.

Thus, the operating parameters of the personal care device may be set automatically, requiring no user input. For example, a user who intends to use the personal care device body will likely be the user who is closest to the personal care device body. A remote device carried by such a user will likely be the remote device which is closest to the personal care device body. The selected remote device may be the remote device of the plurality of remote devices which is closest in distance, or in closest proximity, to the personal care device body. The personal care system may determine the distances of at least one of the plurality of remote devices with respect to the personal care device body, and may determine which of the plurality of remote devices is closest to the personal care device body. The personal care device body may comprise a sensor which is configured to detect the distance of a remote device from the personal care device body. The personal care device body may determine the distance of a remote device based on signal strength of signals emitted between the personal care device body and the remote device. A stronger signal may indicate a smaller distance between the personal care device body and the remote device. A signal may indicate that the personal care device body and a first remote device are in the same room. If the signal indicates that no other remote devices are in the same room, then the first remote device may be the device which is closest in distance to the personal care device body. The personal care system may determine the relative distances between the personal care device body and the plurality of remote devices. For example, the location of each of the personal care device body and the plurality of remote devices may be determined and used to calculate the distances between the personal care device body and each of the remote devices.

Each of the remote devices and the personal care device body may indicate their locations as determined by GPS or the like, and the personal care system may determine the location of each of the remote devices relative to the personal care device body.

A user may indicate which of the remote device is to be selected from the plurality of remote devices. For example, a user may indicate that a remote device is to be selected by opening an application (app) on the remote device. The user may change which device is the selected device. For example, the device which is closest in distance to the personal care device may be initially selected. The user may then indicate that a different device is to be selected, for example via an application on the different remote device.

According to a further aspect the at least one operating parameter may be selected from at least one of the following: an on mode, an off mode, a use mode, an intensity of the operation of the personal care device body, a total use time, a partial use time.

For example, once the remote device is selected, the operating parameter may be determined. The operating parameter may be determined in any of the ways described above. The user may select the operating parameter using an application on the remote device. For example, the user may control the personal care device body using the remote device. The personal care device body may be switched on or off using the remote device. The mode of use may be selected using the remote device. The amount of time which the personal care device body is used for may be selected using the remote device. For example, where the personal care device body is a toothbrush, the quadrant brushing time and/or total brushing time in which the personal care device body is used by the user may be selected. Where the personal care device body is a hairdryer, the use mode, such as the temperature of the hairdryer, and/or an intensity of operation of the hairdryer, such as the power of the motor, may be selected as the at least one operating parameter.

According to a further aspect, the personal care device body may not comprise at least one of: finger or thumb operable control elements in a handle of the personal care device body, finger or thumb operable control elements.

By removing the requirement for buttons or knobs in (a handle of) the personal care device body, the cost of manufacture is reduced. Furthermore, having no finger or thumb operable control elements means that there are fewer ingress paths for water into the personal care device body, which reduces likelihood of failure of the personal care device body. Additionally, there is an increase in freedom of design of inner components of the personal care device body, such as the electronics, as such a design is no longer constrained by the position of control elements which are commonly dictated by user ergonomics.

The control elements may include any of slideable elements, pressable buttons, switches etc.. For example, the control element may be any physical button which is operated by a finger or thumb of a user in normal use.

According to a further aspect, the personal care device body may only be operable via a remote device with which it is paired.

Thus, the personal care device body is operable remotely via the remote device. This allows the personal care device body to be provided with no control elements, as the personal care device body is controlled through the remote device. For example, the operating parameters of the oral care device may be determined by the remote device.

According to a further aspect, the personal care device body may be configured to pair with at least one of the plurality of remote devices when the personal care device body is moved in a predefined motion.

Thus, pairing of a remote device occurs when the personal care device body is moved in a predefined motion. For example, the personal care device body may initiate pairing, or become receptive to pairing, when moved in (substantially) the predefined motion. The predefined motion may be a movement which follows a particular path. The predefined motion may be a set motion which is defined in factory settings of the personal care device. The movement of the personal care device body may be determined using a motion sensor, such as a gyroscope etc.. Any motion may be set as the predefined motion. For example, the personal care device body may be shaken, moved in a circle, figure of eight, or any general predefined path. A remote device may pair with the personal care device body when the remote device is in proximity of the personal care device body and is moved in the predefined motion.

According to a further aspect, the personal care device body may be configured to be switched on by applying pressure to a tool attached to the personal care device body, and/or is switched off by removing pressure from the tool attached to the personal care device body.

For example, the personal care device body may be a toothbrush comprising a brush head (tool). When pressure is applied to the brush head, for example when a user presses the brush against their teeth, the personal care device body may be switched on, or a brushing motion may be activated. When pressure is removed from the brush head, the personal care device body may be switched off, or the brushing motion may be switched off. Alternatively, for example, the personal care device body may be a shaver comprising a shaving head. When pressure is applied to the shaving head, the shaver may be switched on, or a shaving mode may be activated. When pressure is removed from the shaving head, the shaver may be switched off, or the shaving mode may be deactivated.

Switching the personal care device body on and/or off in this manner allows the removal of on/off controls in the handle of a personal care device body, which benefits from the advantages set out above.

According to a further aspect, the personal care device body may be chosen from a group of personal care device bodies comprising: an oral care device body, a toothbrush body, an oral irrigator body, a flossing device body, a hair removal device (epilator, trimmer, shaver) body, skin care device body, grooming device body, hair care device body; and the remote device is chosen from a group of remote devices comprising: a portable device, a battery powered device, an audio device, a computing device, a communications device, a personal media player, a mobile telephone, a games device, a voice controlled device, a tablet, a smartwatch.

The personal care device body may be any personal care device body. The remote device may be a wireless device.

According to a further aspect, the personal care system may comprise the personal care device body and the remote device.

According to a further aspect, there may be provided a computer implemented method, the method comprising: pairing a personal care device body with a plurality of remote devices; selecting a device from the plurality of remote devices; and determining at least one operating parameter of the personal care device body based on the selected device.

According to a further aspect, there may be provided a personal care device body configured to be paired with a plurality of remote devices and to operate according to an operating parameter determined based on a remote device selected from the plurality of remote devices.

According to a further aspect, there may be provided a remote device of a plurality of remote devices which is configured to pair with the personal care device body.

### BRIEF DESCRIPTION OF THE DRAWINGS

Embodiments of the present disclosure may take form in various components and arrangements of components, and in various steps and arrangements of steps. Accordingly, the drawings are for purposes of illustrating the various embodiments and are not to be construed as limiting the embodiments. In the drawing Figs., like reference numerals refer to like elements. In addition, it is to be noted that the Figs. may not be drawn to scale.
Fig. 1 is a schematic diagram which illustrates a personal care system;
Fig. 2 is a schematic diagram which illustrates movement of a personal care device body in a predefined motion;
Fig. 3 is a schematic diagram which illustrates a personal care system;
Fig. 4 is a diagram illustrating an example of a personal care device;
Fig. 5 is a schematic diagram which illustrates a personal care system;
Fig. 6 is a schematic diagram illustrating the operation of a personal care system;
Fig. 7 is a schematic diagram illustrating the selection of a remote device;
Fig. 8 is a schematic diagram illustrating the selection of a remote device; and
Fig. 9 is a flow diagram illustrating a computer implemented method of operating a personal care device.

### DETAILED DESCRIPTION OF EMBODIMENTS

The embodiments of the present disclosure and the various features and advantageous details thereof are explained more fully with reference to the non-limiting examples that are described and/or illustrated in the drawings and detailed in the following description. It should be noted that the features illustrated in the drawings are not necessarily drawn to scale, and features of one embodiment may be employed with other embodiments as the skilled artisan would recognize, even if not explicitly stated herein. Descriptions of well-known components and processing techniques may be omitted so as to not unnecessarily obscure the embodiments of the present disclosure. The examples used herein are intended merely to facilitate an understanding of ways in which the embodiments of the present may be practiced and to further enable those of skill in the art to practice the same. Accordingly, the examples herein should not be construed as limiting the scope of the embodiments of the present disclosure, which is defined solely by the appended claims and applicable law.

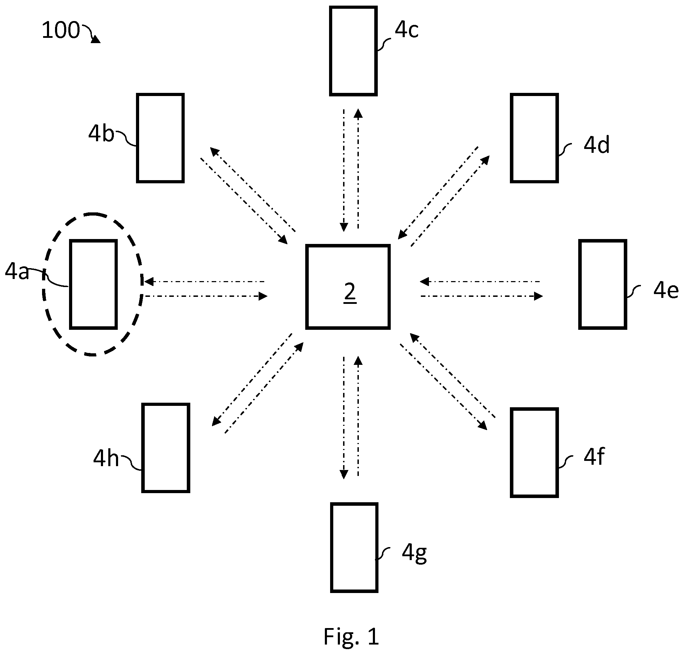

Fig. 1 shows an example of a personal care system 100. The personal care system 100 comprises a personal care device body 2. The personal care system 100 is configured to enable the personal care device body 2 to be paired with a plurality of remote devices 4a-4h. The pairing of the remote devices 4a-4h is indicated by the dotted arrows shown in Fig. 1 representing the potential for communication of information between the plurality of remote devices 4a-4h and the personal care device body 2. In this example, each of the plurality of remote devices 4a-4h are paired to the personal care device body 2 using Bluetooth. Communication between any of the plurality of remote devices 4a-4h and the personal care device may be achieved using any known form of wireless communication, for example, by using electromagnetic waves, radio waves, light, magnetic, or electric fields or the use of sound.

A selected remote device 4a is indicated by the dotted ring shown in Fig. 1. The selected remote device 4a is selected from the plurality of remote devices 4a-4h with which the personal care device body 2 is paired. At least one operating parameter of the personal care device body 2 is determined according to an attribute of the selected remote device 4a, and the personal care device body 2 operates according to the at least one operating parameter.

In an example, the personal care system is configured to determine if at least one previous operating parameter of the personal care device body 2 has been previously determined according to an attribute of the selected remote device. For example, if a remote device has previously been paired with the personal care device body, the operating parameters which were set in the previous pairing are used as the at least one operating parameter. In this example, the previous operating parameters are stored in a memory (not shown) of the personal care device body along with an indication of which remote device each at least one operating parameter is associated with. Alternatively, this information may be stored in the remote device and communicated to the personal care device body.

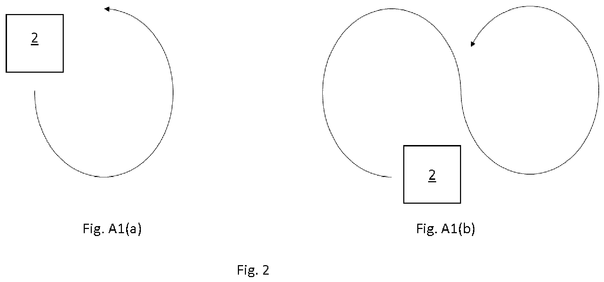

Fig. 2 shows an example of the initiation of pairing of the personal care device body 2 with a remote device 4a-4h. In order to pair the personal care device body 2 with at least one of the plurality of remote devices 4a-4h, in this example the personal care device body 2 is moved in a predetermined motion. The predetermined motion is a particular way in which the personal care device body 2 is moved which indicates to the personal care device body that pairing is to occur. The personal care device body 2 comprises a motion sensor, such as an accelerometer, which is configured to monitor motion of the personal care device body 2. When the personal care device body 2 is moved in a predetermined motion, the motion sensor indicates to the personal care device body 2 that the personal care device body 2 should be enabled to be paired, or indicates to the remote device 4a-4h that pairing should occur. The personal care device body 2 is then paired with the remote device. Fig. 2 shows two examples of such predetermined motions. In Fig. 2(a), the predetermined motion is a substantially circular motion. In Fig. 2(b), the predetermined motion is a Fig. of eight. Any motion may be defined as the predetermined motion, for example, in factory settings of the personal care device body 2. For example, a shaking motion may be used as the predetermined motion.

Alternatively or additionally, the pairing may be initiated by a signal emitted from the personal care device or at least one of the plurality of remote devices, and received by the other of the personal care device or at least one of the plurality of remote devices, indicating that pairing should occur. The signal may be emitted in response to an indication by a user of the personal care device or a remote device.

Any remote devices within a particular radius, for example, 30m, of the personal care device may be paired to the personal care device. The radius may be determined by the range of the personal care device.

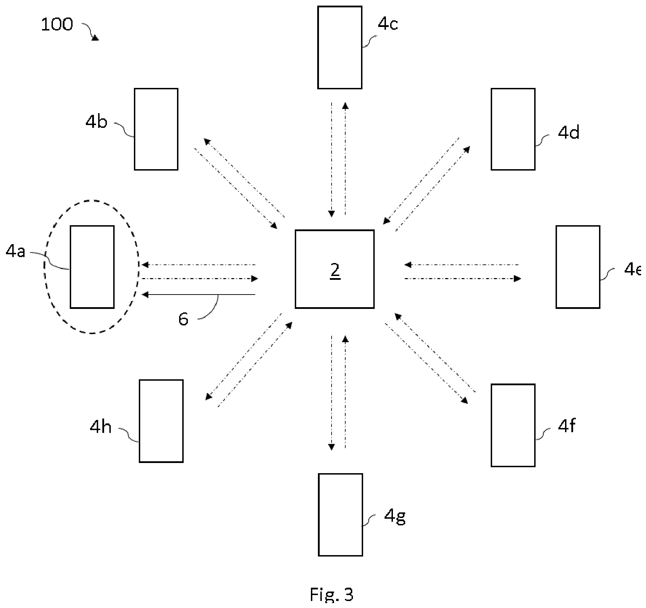

Fig. 3 shows a further example of a personal care system 100. In this example, the personal care device body 2 is configured to transmit information to the selected remote device 4a. The transmission is indicated by the solid arrow 6 shown in Fig. 3. The information may be information about a user, information about use of the personal care device body 2, information about operation of the personal care device body 2, information about use of a tool attached to the personal care device body 2, information about operation of a tool attached to the personal care device body 2, or any combination thereof.

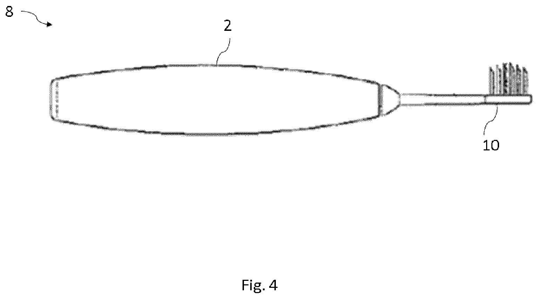

Fig. 4 shows an example of a personal care device 8 comprising a personal care device body 2 and a tool 10. In this example, the personal care device 8 is an oral care device, more specifically a toothbrush. It is noted that, while an oral care device is used as an example of a personal care device, the disclosure herein is not limited to an oral care device, and could apply to any personal care device, such as a hair removal device, a skin care device, a hair care device or any grooming device. The toothbrush comprises a personal care device body 2 (toothbrush body) and a tool 10 (toothbrush head). In this example, the toothbrush head is detachable from the personal care device body 2. However, the tool 10 may be substantially non-detachable from the personal care device body 2, and/or the tool 10 may be integrated into the personal care device body 2. A personal care device may comprise the personal care device body 2 and a tool 10. The operation of the personal care device body 2 may cause a tool of a personal care device to operate in a particular manner based on the operating parameters. The operating parameters may include operations such as turning the personal care device on or off, turning on or off, or changing, a particular mode of operation, e.g. a use mode, selecting or varying the intensity of the operation of the personal care device (body), the amount of time the personal care device (body) is to be used for, or a portion of time thereof.

Where the personal care device body 2 of Fig. 3 is a toothbrush body as shown in Fig. 4, the information may be information about a user, information about use of the toothbrush body, information about operation of the toothbrush body, information about use of a toothbrush head attached to the toothbrush body, information about operation of a toothbrush head attached to the personal care device body, or any combination thereof.

The personal care device body 2 shown in Fig. 4 comprises no finger or thumb operable control elements in a handle of the personal care device body 2. For example, the personal care device body 2 may not comprise any finger or thumb operable control elements, such as buttons, switches, sliders etc.. In this example, the personal care device body 2, and therefore the personal care device including the tool attached to the personal care device, is operable only via a remote device with which it is paired using any of the examples of a personal care system described herein. In an alternative example, the personal care device body 2 is operable via a remote device, but the personal care device, or a mode of operation such as a brushing mode, can be switched on by applying pressure to a tool attached to the personal care device and/or can be switched off by removing pressure from the tool attached to the personal care device body 2. In this example, the tool is provided with a pressure sensor which detects pressure or lack thereof. Alternatively, the personal care device may be operable via finger or thumb operable control elements provided on the personal care device body 2, and via a remote device.

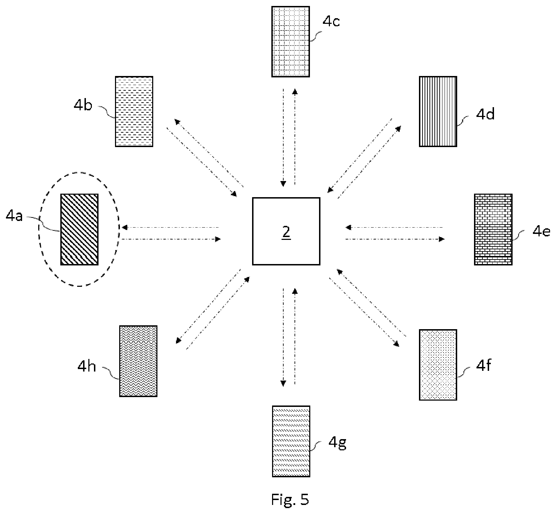

Fig. 5 shows an example of a personal care system in which the identity of the selected remote device 4a determines the at least one operating parameter of the personal care device body 2. As is shown in Fig. 5, each of the plurality of remote devices 4a-4h has a different identity (each identity indicated by a different pattern). The personal care device body 2 determines the identity of the selected remote device 4a. In this example, at least one operating parameter is associated with the identity of the selected remote device 4a. Each of the plurality of remote devices 4a-4h may have a different at least one operating parameter associated with them. Once the identity of the selected remote device 4a is determined by the personal care device body 2, the personal care device body 2 can operate according to the at least one operating parameter associated with the identity of the selected remote device 4a. In this example embodiment, the personal care device body 2 comprises a memory (not shown) configured to store the at least one operating parameter of each of the plurality of remote devices 4a-4h. Upon identifying the selected remote device 4a, the personal care device body 2 accesses the memory to determine the operating parameter(s) of the personal care device body 2 based on the identity of the selected remote device 4a, which in this case is the attribute of the remote device, and operates the personal care device body 2 according to the determined operating parameter(s). Each of the plurality of remote devices 4a-4h may have a default at least one operating parameter which is initially used by the personal care device body 2.

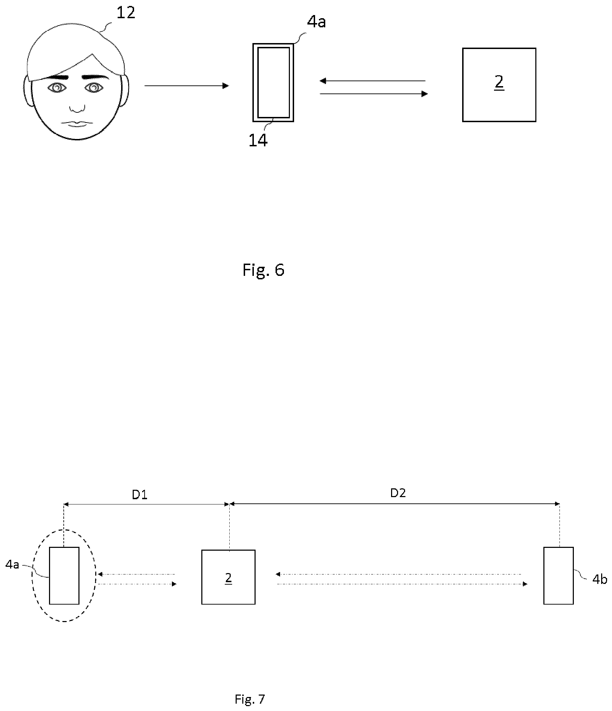

Fig. 6 shows an example of a personal care system in which a user 12 interacts with the selected remote device 4a to input an indication of the at least one operating parameter of the personal care device body 2 via a user interface 14. In this example, the user interface 14 is provided in the remote device and is in the form of an application (app) with which the user can interact. However, the user interface may be provided in the personal care device body 2. The user interface may be in the form of physical buttons or the like, or may be in the form of a touch screen or voice activation. The user 12 inputs at least one operating parameter to the remote device 4a via the user interface 14. The remote device then indicates to the personal care device body 2 the at least one operating parameter, and the personal care device operates according to the at least one operating parameter. The user may indicate the at least one operating parameter during use of the personal care device, for example, while the personal care device is being operated according to an operating parameter. The user may alter the operating parameter. The user may indicate the at least one operating parameter to the remove device via the interface before the selected remote device is paired to the personal care device. Once the personal care device is paired with the selected remote device, the remote device may indicate to the personal care device the at least one operating parameter.

Fig. 7 shows an example of the selection of a remote device from a plurality of remote devices. In Fig. 7, a first remote device 4a and a second remote device 4b are paired with the personal care device body 2, as indicated by the dotted lines. The first remote device 4a is a first distance D1 away from the personal care device body 2. The second remote device 4b is a second distance D2 away from the personal care device body 2. Each distance may be determined using GPS locations of each of the remote devices and the personal care device. The distance from the personal care device may be calculated from a predefined location on the personal care device to a predefined location on each of the remote devices, for example, from the edge of the personal care device to the edge of each remote device, or from the centre of the personal care device to the centre of each remote device. As is shown in this Fig., the first distance D1 is smaller than the second distance D2. Therefore, the first remote device 4a is closer to the personal care device body 2 than the second remote device 4b, so the first remote device 4a is selected as the selected remote device. In this example, the personal care device determines which of the remote devices 4a, 4b is the closest to the personal care device. However, the remote device may be selected by the personal care system. For example, at least one of the plurality of remote devices 4a, 4b may determine which of the remote devices 4a, 4b is closest to the personal care device body 2, and indicate this to the personal care device body 2. The personal care device body 2 may determine which of the remote devices 4a, 4b is closest to the personal care device body 2. Each of the remote devices and the personal care device body 2 may indicate their position, determined by GPS or the like, by wireless communication. At least one of the plurality of remote devices, the personal care device, and/or a computing device (not shown), may determine the closest remote device to the personal care device, and may indicate to any of the remote devices and/or the personal care device which remote device is closest to the personal care device.

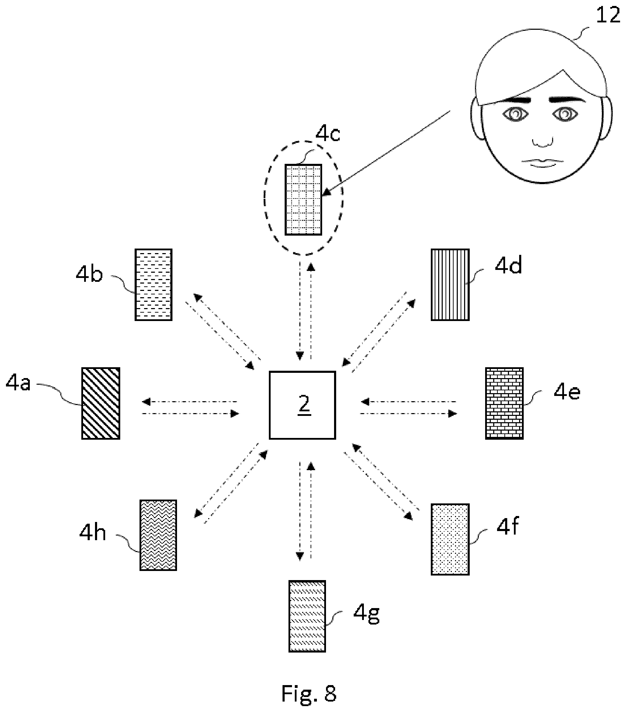

As is shown in Fig. 8, additionally or alternatively, the user indicates which remote device of the plurality of remote devices 4a-4h is the selected remote device. In the example shown in Fig. 8, the user 12 indicates which of the remote devices is to be used to operate the personal care device. In this example, the user indicates which of the remote devices is to be used by opening an application (app) on the remote device which controls the operation of the personal care device body 2. However, the indication may be made once the app is opened by interacting with the app. In this example, the user opens the app on a third remote device 4c. The third remote device 4c then signals to the personal care device body 2 that it is the selected device.

The remote device which is closest in distance to the personal care device body 2 may initially be selected. For example, the process described in relation to Fig. 7 may be used such that the first remote device 4a is initially selected as the selected device. The user may then indicate that the third remote device 4c is to be selected as the selected device.

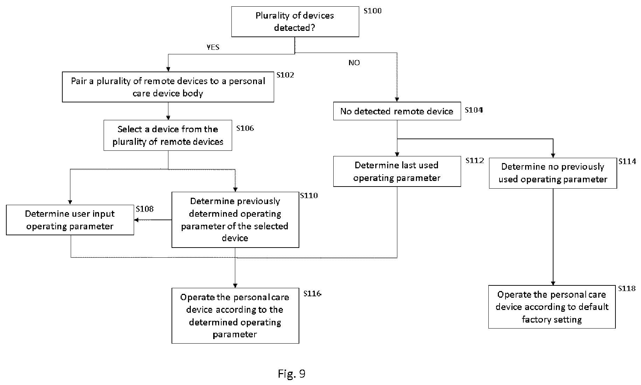

Fig. 9 is a flowchart showing an example of the process of operating the personal care device body 2. In step S100, the personal care device body 2 attempts to detect a plurality of remote devices. If a plurality of remote devices are detected, a plurality of remote devices are paired to the personal care device body 2, S102. A remote device is then selected from the plurality of remote devices, S106. At this point, the operating parameters may be directly obtained by user input, S108, or a previously determined operating parameter of the selected device may be determined S110. The personal care device then operates according to the determined operating parameter, S116. If the previously determined operating parameter is determined in step S110, user input may override the previously determined operating parameter of the selected device and the process may move to step S108 before moving to step S116.

After S100, if no remote device is detected, the personal care device body 2 determines what the last used operating parameter was, S112, and operates the personal care device according to that operating parameter, S116. Alternatively, if no operating parameter has been used, S114, for example, the personal care device has never paired with a remote device, then the personal care device is operated according to default factory settings, S118.

In any of the above examples, any of the remote devices may comprise at least one transmitter for transmitting signals, at least one receiver for receiving signals, at least one memory for storing information, at least one processor for processing information, at least one sensor, and/or at least one microphone. The personal care device may comprise at least one transmitter for transmitting signals, at least one receiver for receiving signals, at least one memory for storing information, at least one processor for processing information, at least one sensor, at least one means for carrying out an operating parameter, at least one means for operating a tool, at least one sensor, and/or at least one microphone.

In any of the above aspects, the various features may be implemented in hardware, or as software modules running on one or more processors. Features of one aspect may be applied to any of the other aspects.

The invention also provides a computer program or a computer program product for carrying out any of the methods described herein, and a computer readable medium having stored thereon a program for carrying out any of the methods described herein. A computer program embodying the invention may be stored on a computer-readable medium, or it could, for example, be in the form of a signal such as a downloadable data signal provided from an Internet website, or it could be in any other form.

It is understood that the embodiments of the present disclosure are not limited to the particular methodology, protocols, devices, apparatus, materials, applications, etc., described herein, as these may vary. It is also to be understood that the terminology used herein is used for the purpose of describing particular embodiments only, and is not intended to be limiting in scope of the embodiments as claimed. It must be noted that as used herein and in the appended claims, the singular forms "a," "an," and "the" include plural reference unless the context clearly dictates otherwise.

Unless defined otherwise, all technical and scientific terms used herein have the same meanings as commonly understood by one of ordinary skill in the art to which the embodiments of the present disclosure belong. Preferred methods, devices, and materials are described, although any methods and materials similar or equivalent to those described herein may be used in the practice or testing of the embodiments.

Although only a few exemplary embodiments have been described in detail above, those skilled in the art will readily appreciate that many modifications are possible in the exemplary embodiments without materially departing from the novel teachings and advantages of the embodiments of the present disclosure. The above-described embodiments of the present invention may advantageously be used independently of any other of the embodiments or in any feasible combination with one or more others of the embodiments.

Accordingly, all such modifications are intended to be included within the scope of the embodiments of the present disclosure as defined in the following claims. In the claims, means-plus-function clauses are intended to cover the structures described herein as performing the recited function and not only structural equivalents, but also equivalent structures.

In addition, any reference signs placed in parentheses in one or more claims shall not be construed as limiting the claims. The word "comprising" and "comprises," and the like, does not exclude the presence of elements or steps other than those listed in any claim or the specification as a whole. The singular reference of an element does not exclude the plural references of such elements and vice-versa. One or more of the embodiments may be implemented by means of hardware comprising several distinct elements. In a device or apparatus claim enumerating several means, several of these means may be embodied by one and the same item of hardware. The mere fact that certain measures are recited in mutually different dependent claims does not indicate that a combination of these measures cannot be used to an advantage.

## Claims

1. A personal care system (100) comprising a personal care device body (2), wherein the personal care system (100) is configured to enable:
the personal care device body (2) to be paired with a plurality of remote devices (4a-4h);
when the personal care device body is paired with the plurality of remote devices (4a-4h), a remote device (4a) to be selected from the plurality of remote devices (4a-4h) with which the personal care device body (2) is paired; and
at least one operating parameter of the personal care device body (2) to be determined according to an attribute of the selected remote device (4a);
and wherein the personal care device body (2) is configured to operate according to the at least one operating parameter.

2. The personal care system (100) as claimed in claim 1, wherein the personal care device body (2) is configured to transmit information to the selected remote device (4a), the information being at least one of: information about a user (12), information about use of the personal care device body (2), information about operation of the personal care device body (2), information about use of a tool (10) attached to the personal care device body (2), information about operation of a tool (10) attached to the personal care device body (2).

3. The personal care system (100) as claimed in any preceding claim, wherein the attribute includes an identity of the selected remote device (4a) which determines the at least one operating parameter of the personal care device body (2).

4. The personal care system (100) as claimed in any preceding claim, wherein
the attribute includes an indication of the at least one operating parameter input by a user (12) to an interface, and wherein
the interface is comprised in the remote device (4a).

5. The personal care system (100) as claimed in any preceding claim, wherein the remote device (4a) is selected based on at least one of: the remote device (4a) of the plurality of remote devices (4a-4h) which is closest in distance to the personal care device body (2), input by a user (12) which indicates which remote device (4a) is to be selected.

6. The personal care system (100) as claimed in any preceding claim, wherein the personal care system (100) is configured to determine if at least one previous operating parameter of the personal care device body (2) has been previously determined according to an attribute of the selected remote device (4a), and when at least one previous operating parameter has been previously determined, to determine the at least one operating parameter based on the previous operating parameter.

7. The personal care system (100) as claimed in any preceding claim, wherein the at least one operating parameter is selected from at least one of the following: an on mode, an off mode, a use mode, an intensity of the operation of the personal care device body (2), a total use time, a partial use time.

8. The personal care system (100) as claimed in any preceding claim, wherein the personal care device body (2) does not comprise at least one of: finger or thumb operable control elements in a handle of the personal care device body (2), finger or thumb operable control elements.

9. The personal care system (100) as claimed in any preceding claim, wherein the personal care device body (2) is only operable via a remote device (4a-4h) with which it is paired.

10. The personal care system (100) as claimed in any preceding claim, wherein the personal care device body (2) is configured to pair with at least one of the plurality of remote devices (4a-4h) when the personal care device body (2) is moved in a predefined motion.

11. The personal care system (100) as claimed in any preceding claim, wherein the personal care device body (2) is configured to be switched on by applying pressure to a tool (10) attached to the personal care device body (2), and/or is switched off by removing pressure from the tool (10) attached to the personal care device body (2).

12. The personal care system (100) as claimed in any preceding claim, wherein
the personal care device body (2) is chosen from a group of personal care device (8) bodies comprising: an oral care device body, a toothbrush body, an oral irrigator body, a flossing device body, a hair removal device body, skin care device body, grooming device body, hair care device body; and
at least one of the plurality of remote devices (4a-4h) is chosen from a group of remote devices comprising: a portable device, a battery powered device, an audio device, a computing device, a communications device, a personal media player, a mobile telephone, a games device, a voice controlled device, a tablet.

13. A personal care device body (2) configured to be paired with a plurality of remote devices (4a-4h) and to operate according to an operating parameter determined according to an attribute of a remote device (4a) selected from the plurality of remote devices (4a-4h).

14. A remote device of a plurality of remote devices (4a-4h) which is configured to pair with the personal care device body (2) of claim 13.

15. A computer implemented method, the method comprising:
pairing a personal care device body (2) with a plurality of remote devices (4a-4h);
selecting a remote device (4a) from the plurality of remote devices (4a-4h); and
determining at least one operating parameter of the personal care device body (2) according to an attribute of the selected remote device (4a).
